# EUROPEAN PATENT APPLICATION

(11) **EP 3 251 516 A1**
(43) Date of publication of application: **06.12.2017**
(21) Application number: 16171945.5
(22) Date of filing: 30.05.2016
(51) Int. Cl.: A23D 7/00, A61K 31/202, A23L 33/12

(54) **NUTRITIONAL COMPOSITION**

(71) Applicant: Instituto Nacional de Medicina Genomica (INMEGEN), 14610 Mexico (MX)
(72) Inventor: TEJERO BARRERA, Elizabeth Maria, 03020 MEXICO (MX); BINIA, Aristea, 1700 Fribourg (CH); SILVA ZOLEZZI, Irma, 1084 Carrouge (CH); KUSSMANN, Martin, 1066 Epalinges (CH)
(74) Representative: Couzens, Patrick John

(57) **Abstract**

The present invention provides a nutritional composition comprising an omega-3 polyunsaturated fatty acid as an active agent for use in reducing triglyceride levels in a subject having a peroxisome proliferator-activated receptor-alpha (PPARα) X162V genetic variant; where X is leucine or valine.

SEQUENCE LISTING

<110> INSTITUTO NACIONAL DE MEDICINA GENÃ\223MICA

<120> Nutritional composition

<130> 14758-EP-EPA

<160> 2

<170> BiSSAP 1.2

<210> 1
<211> 472
<212> PRT
<213> Homo sapiens

<400> 1
Met Val Asp Thr Glu Ser Pro Leu Cys Pro Leu Ser Pro Leu Glu Ala
1               5                   10                  15
Gly Asp Leu Glu Ser Pro Leu Ser Glu Glu Phe Leu Gln Glu Met Gly
            20                  25                  30
Asn Ile Gln Glu Ile Ser Gln Ser Ile Gly Glu Asp Ser Ser Gly Ser
        35                  40                  45
Phe Gly Phe Thr Glu Tyr Gln Tyr Leu Gly Ser Cys Pro Gly Ser Asp
    50                  55                  60
Gly Ser Val Ile Thr Asp Thr Leu Ser Pro Ala Ser Ser Pro Ser Ser
65                  70                  75                  80
Val Thr Tyr Pro Val Val Pro Gly Ser Val Asp Glu Ser Pro Ser Gly
                85                  90                  95
Ala Leu Asn Ile Glu Cys Arg Ile Cys Gly Asp Lys Ala Ser Gly Tyr
            100                 105                 110
His Tyr Gly Val His Ala Cys Glu Gly Cys Lys Gly Phe Phe Arg Arg
        115                 120                 125
Thr Ile Arg Leu Lys Leu Val Tyr Asp Lys Cys Asp Arg Ser Cys Lys
    130                 135                 140
Ile Gln Lys Lys Asn Arg Asn Lys Cys Gln Tyr Cys Arg Phe His Lys
145                 150                 155                 160
Cys Leu Ser Val Gly Met Ser His Asn Ala Ile Arg Phe Gly Arg Met
                165                 170                 175
Pro Arg Ser Glu Lys Ala Lys Leu Lys Ala Glu Ile Leu Thr Cys Glu
            180                 185                 190
His Asp Ile Glu Asp Ser Glu Thr Ala Asp Leu Lys Ser Leu Ala Lys
        195                 200                 205
Arg Ile Tyr Glu Ala Tyr Leu Lys Asn Phe Asn Met Asn Lys Val Lys
    210                 215                 220
Ala Arg Val Ile Leu Ser Gly Lys Ala Ser Asn Asn Pro Pro Phe Val
225                 230                 235                 240
Ile His Asp Met Glu Thr Leu Cys Met Ala Glu Lys Thr Leu Val Ala
                245                 250                 255
Lys Leu Val Ala Asn Gly Ile Gln Asn Lys Glu Ala Glu Val Arg Ile
            260                 265                 270
Phe His Cys Cys Gln Cys Thr Ser Val Glu Thr Val Thr Glu Leu Thr
        275                 280                 285
Glu Phe Ala Lys Ala Ile Pro Gly Phe Ala Asn Leu Asp Leu Asn Asp
    290                 295                 300
Gln Val Thr Leu Leu Lys Tyr Gly Val Tyr Glu Ala Ile Phe Ala Met
305                 310                 315                 320
Leu Ser Ser Val Met Asn Lys Asp Gly Met Leu Val Ala Tyr Gly Asn
                325                 330                 335
Gly Phe Ile Thr Arg Glu Phe Leu Lys Ser Leu Arg Lys Pro Phe Cys
            340                 345                 350

Asp Ile Met Glu Pro Lys Phe Asp Phe Ala Met Lys Phe Asn Ala Leu
        355                 360                 365
Glu Leu Asp Asp Ser Asp Ile Ser Leu Phe Val Ala Ala Ile Ile Cys
    370                 375                 380
Cys Gly Asp Arg Pro Gly Leu Leu Asn Val Gly His Ile Glu Lys Met
385                 390                 395                 400
Gln Glu Gly Ile Val His Val Leu Arg Leu His Leu Gln Ser Asn His
                405                 410                 415
Pro Asp Asp Ile Phe Leu Phe Pro Lys Leu Leu Gln Lys Met Ala Asp
            420                 425                 430
Leu Arg Gln Leu Val Thr Glu His Ala Gln Leu Val Gln Ile Ile Lys
        435                 440                 445
Lys Thr Glu Ser Asp Ala Ala Leu His Pro Leu Leu Gln Glu Ile Tyr
    450                 455                 460
Arg Asp Met Tyr Ser Asn Pro Ser
465                 470

<210> 2
<211> 505
<212> PRT
<213> Homo sapiens

<400> 2

## Description

### FIELD OF THE INVENTION

The present invention relates to compositions and methods useful for reducing triglyceride levels in a subject.

### BACKGROUND TO THE INVENTION

The human body utilises lipids in a variety of physiological and metabolic processes. Accordingly, lipids may be found circulating in the bloodstream or stored in adipose tissue. The most abundant lipids are triglycerides (also known as triacylglycerides or TAG), which are composed of glycerol and three fatty acid moieties.

While a minimum level of circulating TAG is required for normal physiology, an elevated level of TAG, known as hypertriglyceridemia, is associated with an increased risk of cardiovascular disease, in particular atherosclerosis. In this regard, the Adult Treatment Panel III *(*JAMA 2001 285: 2486-97) of the National Cholesterol Education Program has proposed four ranges, which are now in common use for assessing the risk associated with hypertriglyceridemia - see Table 1. These strata are in line with a 10-year follow up meta-analysis study, which showed that a 1 mmol/L increase in TAG level increased the risk of cardiovascular disease by 32% in men and by 76% in women (Hokanson and Austin, J Cardiovasc Risk 1996 3: 213-219). Additionally, individuals with severely high triglyceride levels, i.e. >10 mmol/L are also at increased risk of acute pancreatitis (Yuan et al., CMAJ 2007 176(8); 1113).

**Table 1**

| **Level (mg/dL)** | **Level (mmol/L)** | **Interpretation** |
|---|---|---|
| <150 | <1.70 | Normal range |
| 150-199 | 1.70-2.25 | Borderline high |
| 200-499 | 2.26-5.65 | High |
| >500 | >5.65 | Very high |

Where an elevated TAG level is caused by an underlying condition, this disorder is referred to as secondary hypertriglyceridemia. Such underlying conditions or lifestyle factors include obesity, diabetes, hypothyroidism, kidney disease, excess calorific intake, and excess alcohol consumption. A number of medications are also known to cause or contribute to hypertriglyceridemia, including corticosteroids, tamoxifen, antihypertensives, antiretrovirals and second-generation antipsychotic medications. Alternatively, individuals that exhibit elevated triglyceride levels but no underlying cause are said to have primary hypertriglyceridemia (Yuan et al., CMAJ 2007 176(8); 1113).

Given the association between hypertriglyceridemia and cardiovascular disease, there has been considerable interest in drugs or methods to prevent or treat elevated TAG levels *in vivo.* To this end, several studies have been conducted to investigate the link between dietary lipid composition and blood plasma TAG levels. Of these, a number have reported that omega-3 polyunsaturated fatty acid (omega-3 PUFA) supplementation can decrease blood plasma TAG concentration. This phenomenon may be mediated by omega-3 PUFA binding induced activation of peroxisome proliferator-activated receptors α (PPARα) and γ (PPARy). Following ligand-induced activation, PPARα and PPARγ each heterodimerize with retinoid-X receptor α (RXRα) and can then bind to a peroxisome proliferator response element (PPRE) sequence located within a target gene promoter region, thereby initiating transcription. By acting in this way, transcription factors PPARα and PPARγ are involved in the regulation of many genes involved in lipid storage and metabolism, such as lipoprotein lipase or LDL receptor (AlSaleh et al., Proc Nutr Soc 2012 71: 141-153).

PPARγ isoform 2 (PPARy2) is expressed in adipose tissue and accordingly, a number of studies have investigated the association between obesity (and related disorders) and a common PPARy2 single nucleotide polymorphism (SNP) which produces a PPARy2 Pro12Ala variant where proline at position 12 of PPARy2 is substituted by an alanine residue. The frequency of the Pro12Ala variant ranges from between 1 and 39%, depending on ethnic group (1000 Genome Project, Release 16 Oct 2014, http://browser.1000genomes.org). For example, in European ancestry populations Pro12Ala variant frequencies as high as 20% have been reported (Aldhoon et al., Folia Biol (Praha) 2010 56(3): 116-123). Notably, the alanine variant at position 12 of PPARy2 has been shown to have decreased binding affinity for PPRE and reduced transcriptional activation ability (Deeb et al., Nat Genet 1998 20: 284-287).

A recent study in overweight individuals (having an average BMI of 26.5) showed that those who carried the PPARγ2 Pro12Ala (P12A) genetic variant demonstrated a greater decrease in plasma TAG levels following omega-3 PUFA supplementation than that which was observed in Pro12Pro homozygous individuals (P12P) (Lindi et al., MGG 2003 79: 52-60).

Additionally, AlSaleh et al. (Proc Nutr Soc 2012 71: 141-153) reported that, as the ratio of dietary PUFA to saturated fatty acids increased, P12A carriers demonstrated a significant trend towards the reduction of plasma TAG levels. This trend was not replicated in P12P individuals.

PPARα is more widely expressed than PPARy2 and has been shown to regulate gene expression in diverse tissues such as the liver, skeletal muscle, heart, kidney, as well as in the adipose tissue. A widely studied genetic variant of PPARα is the Leu162Val (L162V)variant, where a leucine residue at position 162 of PPARα is substituted by a valine, although the effect of this amino acid change on the affinity of the PPARα-PPRE binding interaction and target gene transcriptional activation remains unclear. Within the European population Leu162Val variant frequencies of between 1 and 14%have been reported(1000 Genome Project, Release 16 Oct 2014, http://browser.1000genomes.org).

Tai *et al.* showed that, where dietary PUFA intake was greater than 8%, plasma TAG levels observed in Leu162Val variant carriers were 4% lower than those of L162L homozygotes (Tai et al., J Nutr 2005 135: 397-403). However, it was concluded that the interaction between PUFA intake and the Leu162Val variant was only significant for omega-6 PUFA supplementation. In contrast, the interaction between omega-3 PUFA supplementation and the response between the L162L homozygotes and Leu162Val variant carriers in terms of plasma TAG levels was not significant.

There remains a need for more robust and targeted methods for reducing TAG levels *in vivo.*

### SUMMARY OF THE INVENTION

The present inventors have surprisingly found that individuals carrying a PPARα X162V genetic variant, where X is leucine or valine, respond well to omega-3 PUFA nutritional supplementation. In particular, individuals with a PPARα X162V genetic variant had a greater reduction in fasting TAG levels following omega-3 PUFA nutritional supplementation compared to individuals homozygous for PPARα Leu162Leu (i.e. PPARα L162L).

The inventors have also surprisingly found that this response is even greater in subjects having both the PPARα X162V genetic variant and a PPARy2 X'12A genetic variant; where X' is proline or alanine.

Thus, in a first aspect the present invention provides an omega-3 PUFA or a nutritional composition comprising an omega-3 PUFA as an active agent for use in reducing triglyceride levels in a subject having a PPARα X162V genetic variant; where X is leucine or valine.

The subject may suffer from a condition associated with elevated triglyceride levels.

The condition associated with elevated triglyceride levels may be selected from hypertriglyceridemia, hyperlipidaemia, dyslipidaemia, cardiovascular disease, atherosclerosis, atheroma, acute pancreatitis, insulin resistance, prediabetes, type 2 diabetes, metabolic syndrome, hypertension and non-alcoholic fatty liver disease.

In a further aspect the present invention provides an omega-3 PUFA or a nutritional composition comprising an omega-3 PUFA as an active agent for use in reducing triglyceride levels to prevent or treat a condition associated with elevated triglyceride levels in a subject having a PPARα X162V genetic variant; where X is leucine or valine.

In a preferred embodiment the subject also has a PPARy2 X'12A genetic variant; where X' is proline or alanine.

The subject may be administered, for example, from about 500 to about 10000 mg omega-3 PUFA per day, preferably from about 1500 to about 3000 mg omega-3 PUFA per day.

In one embodiment, the omega-3 PUFA comprises eicosapentaenoic acid (EPA) and/or docosahexanoic acid (DHA).

The omega-3 PUFA may comprise an EPA to DHA ratio from about 0.5:1 to about 5:1, for example about 0.5:1, 1:1, 1.5:1, 2:1, 2.5:1, 3:1 or 5:1 wt/wt; preferably about 2.5:1 wt/wt.

The subject may be administered, for example, from about 500 to about 4000 mg EPA and from about 500 to about 4000 mg DHA per day, preferably from about 1000 to about 3000 mg EPA and from about 500 to about 1000 mg DHA per day.

The nutritional composition may be in the form of a foodstuff, for example a human food stuff. The nutritional composition may be in the form of a complete nutritional product.

The nutritional composition may be in the form of a capsule, a tablet, a powdered form, a freeze-dried product or an oil-in-water emulsion.

In a further aspect the present invention relates to a method for reducing triglyceride levels, or preventing or treating a condition associated with elevated triglyceride levels by reducing triglyceride levels in a subject comprising administering an omega-3 PUFA or a nutritional composition comprising an omega-3 PUFA as an active agent to the subject; wherein the subject has been identified as having a PPARα X162V genetic variant; where X is leucine or valine.

Preferably, the subject also has a proliferator-activated receptor-gamma 2 (PPARy2) X'12A genetic variant; where X' is proline or alanine.

In another aspect, the present invention relates to a method for reducing triglyceride levels, or preventing or treating a condition associated with elevated triglyceride levels by reducing triglyceride levels in a subject comprising the steps of:
i) determining whether the subject is positive for a PPARα X162V genetic variant; and
ii) administering an omega-3 PUFA or a nutritional composition comprising an omega-3 PUFA as an active agent to a subject identified as having the PPARα X162V genetic variant; where X is leucine or valine.

Preferably, the method also comprises the step of determining whether the subject also has a PPARy2 X'12A genetic variant; where X' is proline or alanine.

In a further aspect the present invention provides the use of an omega-3 PUFA in the preparation of a nutritional product for reducing triglyceride levels, or preventing or treating a condition associated with elevated triglyceride levels by reducing triglyceride levels in a subject, wherein the subject has a PPARα X162V genetic variant; where X is leucine or valine.

Preferably, the subject also has a PPARy2 X'12A genetic variant; where X' is proline or alanine.

### DESCRIPTION OF THE FIGURES

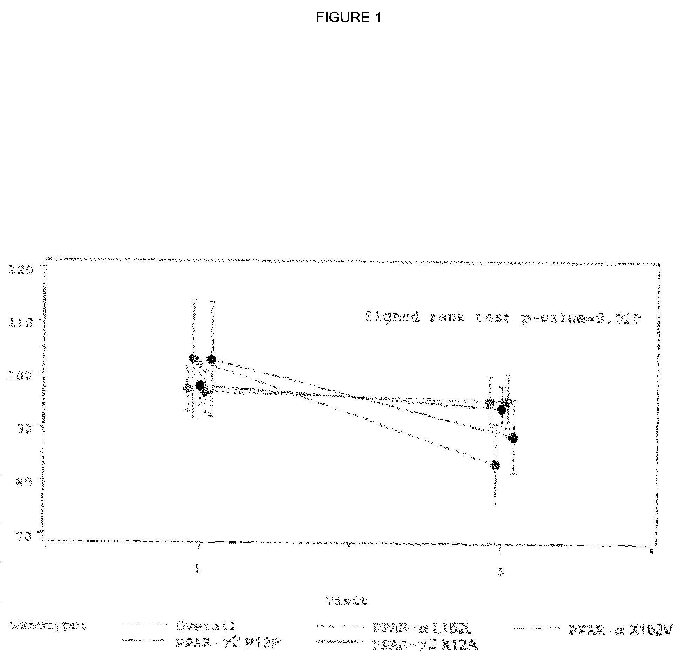

**Figure 1** **-** Omega-3 PUFA supplementation decreases triglyceride levels in subjects having the PPARα L162V and PPARy2 P12A genetic variants. Baseline and week six fasting triglyceride levels are shown for the following subject groups: overall (solid black); PPARα L162L (short dashed green); PPARα X162V (dashed red); PPARy2 P12P (long dashed green) and PPARy2 X21A (long dashed blue).

### DETAILED DESCRIPTION OF THE INVENTION

### TRIGLYCERIDES

The nutritional compositions comprising an omega-3 PUFA described herein are used to reduce triglyceride levels in a subject with a PPARα X162V genetic variant; where X is leucine or valine.

In a preferred embodiment, the triglyceride levels are fasting triglyceride levels.

Triglycerides (also referred to as triacyglycerides or TAGs) are composed of glycerol and three fatty acid molecules. The fatty acid molecules are linked to the glycerol moiety via ester bonds.

TAG are the most abundant lipids present in the circulatory system. Dietary TAG are broken down to glycerol and free fatty acids (FFA) in the gastrointestinal tract by the action of pancreatic lipases. Some short chain FFA may pass directly into the circulatory system, however the majority of FFA are re-esterified to glycerol prior to entering the blood plasma. In order to facilitate their emulsification and transport, TAG are typically bound within lipoprotein assemblies, such as chylomicrons. Such TAG may be released from chylomicrons and broken down by the action of lipoprotein lipases, the resultant FFA may then be used as a substrate for mitochondrial β-oxidation, thereby acting as a source of energy. Alternatively, TAG-derived FFA may be packaged into VLDL by the liver or reconverted into TAG for storage in adipose tissue.

Low levels of TAG are typically associated with good health. Elevated TAG levels are associated with an increased risk of cardiovascular diseases, e.g. atherosclerosis or atheroma (and associated conditions such as heart disease, heart attack and stroke), and acute pancreatitis.

Elevated TAG levels may be associated with an underlying disorder. By way of example, conditions associated with elevated levels of TAG include obesity, diabetes, hypothyroidism, and kidney disease.

Elevated TAG levels may also be cause by lifestyle factors, such as excess calorific intake or excess alcohol consumption.

Fasting TAG levels are typically expressed as milligrams per decilitre (mg/dL). Typically a subject is instructed to fast for at least 8 hours before plasma or serum TAG levels are determined. Levels of TAG may be determined by methods which are well known in the art. For example, serum TAG may be measured enzymatically in a series of coupled reactions in which TAG are hydrolysed to produce glycerol. The glycerol is then oxidized thereby generating H₂O₂, which may be detected in an enzyme-linked colorimetric reaction.

The term "reducing", as used herein is synonymous with terms such as decreasing and lowering. The term "levels", as used herein, refers to any measure of abundance such as amount or concentration. Thus, the present compositions and methods are useful for decreasing TAG levels in a subject.

Reducing TAG levels may mean that TAG levels are reduced by at least 5%, at least 7%, at least 10%, at least 20%, at least 30%, at least 40%, at least 50%, at least 60% or at least 70% compared to the TAG level prior to the administration of a nutritional composition comprising an omega-3 PUFA.

TAG levels are typically determined in blood serum or plasma. Techniques for collecting blood samples and separating blood fractions are well known in the art. For instance, vena blood samples can be collected from patients using a needle and deposited into plastic tubes. The collection tubes may, for example, contain spray-coated silica and a polymer gel for serum separation. Serum can be separated by centrifugation at 1300 RCF for 10 min at room temperature and stored in small plastic tubes at -80°C.

In one embodiment, the present invention relates to the use of an omega-3 PUFA or a nutritional composition comprising an omega-3 PUFA for reducing TAG levels in a subject suffering from a condition associated with elevated TAG levels. Such conditions include, for example, hypertriglyceridemia, hyperlipidaemia, dyslipidaemia, cardiovascular disease, atherosclerosis, atheroma, acute pancreatitis, type 2 diabetes, metabolic syndrome, hypertension and non-alcoholic fatty liver disease.

In one embodiment, the present invention relates to the use of an omega-3 PUFA or a nutritional composition comprising an omega-3 PUFA for the prevention and/or treatment of a condition associated with elevated TAG levels.

The prevention of a condition associated with elevated TAG levels relates to the prophylactic use of an omega-3 PUFA or a nutritional composition as described herein. Accordingly, the omega-3 PUFA or nutritional composition may be administered to a subject who has not yet developed a condition and/or who is not showing any symptoms of the condition to prevent or impair the cause of condition or to reduce or prevent development of at least one symptom associated with the condition.

### PPARα

Peroxisome proliferator-activated receptor α (PPARα) is a member of the nuclear hormone receptor superfamily. PPARα is expressed in variety of tissues, including liver, kidney, heart and skeletal muscle. It is known to be involved in regulating the expression of genes related to fatty acid uptake, lipid and carbohydrate metabolism and mitochondrial *β*-oxidation.

Following ligand-induced activation, PPARα heterodimerises with retinoid-X receptor α (RXRα) and can then bind to a peroxisome proliferator response element (PPRE) sequence located within a target gene promoter region, thereby initiating transcription.

Endogenous PPARα ligands include monounsaturated fatty acids, PUFA and eicosanoids (Lefebvre et al., Proc Natl Acad Sci USA 1997; 94: 4318-4323). Exogenous PPARα ligands include lipid-lowering drugs such as fibrate and fenofibrate.

An example human PPARα protein is the human PPARα protein having the UniProtKB accession number Q07869. This exemplified sequence is 468 amino acids in length and is shown as SEQ ID NO: 1.
SEQ ID NO: 1 (PPARα amino acid sequence)

MVDTESPLCPLSPLEAGDLESPLSEEFLQEMGNIQEISQSIGEDSSGSFGFTEYQYLGSCPGSDGSVI
TDTLSPASSPSSVTYPVVPGSVDESPSGALNIECRICGDKASGYHYGVHACEGCKGFFRRTIRLKLVY
DKCDRSCKIQKKNRNKCQYCRFHKCLSVGMSHNAIRFGRMPRSEKAKLKAEILTCEHDIEDSETADLK
SLAKRIYEAYLKNFNMNKVKARVILSGKASNNPPFVIHDMETLCMAEKTLVAKLVANGIQNKEAEVRI
FHCCQCTSVETVTELTEFAKAIPGFANLDLNDQVTLLKYGVYEAIFAMLSSVMNKDGMLVAYGNGFIT
REFLKSLRKPFCDIMEPKFDFAMKFNALELDDSDISLFVAAIICCGDRPGLLNVGHIEKMQEGIVHVL
RLHLQSNHPDDIFLFPKLLQKMADLRQLVTEHAQLVQIIKKTESDAALHPLLQEIYRDMYSNPS

A widely studied variant in PPARα is the Leu162Val variant in which the leucine at position 162 is substituted by a valine residue (this corresponds to position 162 of SEQ ID NO: 1).

This mutation is caused by the rs1800206 single nucleotide polymorphism (SNP) at codon 162 of PPARα-specific exon 5 (Leu162Val, CTT→GTT).

According to the present invention, the subject has a PPARα X162V genetic variant, where X is leucine or valine. Thus, X is a leucine or valine residue encoded by a first PPARα allele and V is a valine residue encoded by a second PPARα allele. Accordingly, the subject may be homozygous or heterozygous for the valine residue at position 162 of PPARα and may therefore have a PPARα L162V or V162V genotype.

### PPARγ2

In a preferred embodiment of the present invention, the subject also has a PPARy2 X'12A genetic variant; where X' is proline or alanine.

Peroxisome proliferator-activated receptor γ (PPARγ) is a member of the nuclear hormone receptor superfamily. It is known to be involved in regulating the expression of genes related to inflammation, adipose cell differentiation, atherosclerosis and metabolism. The major ligands for PPARγ are PUFA and prostanoids. Like PPARα, following ligand-induced activation, PPARγ heterodimerises with RXRα and can then bind to a PPRE sequence located within a target gene promoter region, thereby initiating transcription.

Four subtypes of PPARγ mRNA transcribed from different promoters give rise to two different PPARγ proteins. The PPARy2 protein is exclusively expressed in adipose tissue. An example human PPARy2 protein is the human PPARy2 protein having the UniProtKB accession number P37231. This exemplified sequence is 505 amino acids in length and is shown as SEQ ID NO: 2.
SEQ ID NO: 2 (PPARγ2 amino acid sequence)

The most widely studied variant in PPARy2 is the Pro12Ala variant in which the proline at position 12 is mutated to an alanine residue (this corresponds to position 12 of SEQ ID NO: 2). This mutation is caused by the rs1801282 single nucleotide polymorphism (SNP) at codon 12 of PPARγ2-specific exon B (Pro12Ala, CCA→GCA).

Thus, in a preferred embodiment of the present invention, the subject also has a PPARy2 X'12A genetic variant, where X' is proline or alanine. Thus, X' is a proline or alanine residue encoded by a first PPARy2 allele and A is an alanine residue encoded by a second PPARy2 allele. Accordingly, the subject may be homozygous or heterozygous for the alanine residue at position 12 of PPARy2 and may therefore have a PPARy2 P12A or A12A genotype.

### DETECTING GENETIC VARIANTS

Genetic variants may be detected using a wide range of techniques and methods which are well known in the art.

PPARα and/or PPARy2 genetic variants may be detected by detecting the SNPs associated with the PPARα Leu162Val variant and/or PPARy2 Pro12Ala variant.

### Detection of alleles

Nucleic acids obtained from a sample can be genotyped to identify the particular allele present for a marker locus. A sample of sufficient quantity to permit direct detection of marker alleles from the sample may be obtained.

Alternatively, a smaller sample is obtained from the subject and the nucleic acids are amplified prior to detection. Optionally, the nucleic acid sample is purified (or partially purified) prior to detection of the marker alleles.

Examples of allele detection methods are given below:

### Allele Specific PCR

Allele-specific PCR differentiates between target regions differing in the presence of absence of a variation or polymorphism. PCR amplification primers are chosen based upon their complementarity to the target sequence, such as a sequence disclosed herein. The primers bind only to certain alleles of the target sequence.

### Allele Specific Oligonucleotide Screening Methods

Further screening methods employ the allele-specific oligonucleotide (ASO) screening methods (e.g. see Saiki et al., Nature 324:163-166, 1986).

Oligonucleotides with one or more base pair mismatches are generated for any particular allele. ASO screening methods detect mismatches between one allele in the target genomic or PCR amplified DNA and the other allele, showing decreased binding of the oligonucleotide relative to the second allele (i.e. the other allele) oligonucleotide. Oligonucleotide probes can be designed that under low stringency will bind to both polymorphic forms of the allele, but which at high stringency, bind to the allele to which they correspond. Alternatively, stringency conditions can be devised in which an essentially binary response is obtained, i.e., an ASO corresponding to a variant form of the target gene will hybridize to that allele, and not to the wild type allele.

### Ligase Mediated Allele Detection Method

Ligase can also be used to detect point mutations, such as the SNPs in a ligation amplification reaction (e.g. as described in Wu et al., Genomics 4:560-569, 1989). The ligation amplification reaction (LAR) utilizes amplification of specific DNA sequence using sequential rounds of template dependent ligation (e.g. as described in Wu, supra, and Barany, Proc. Nat. Acad. Sci. 88:189-193, 1990).

### Denaturing Gradient Gel Electrophoresis

Amplification products generated using the polymerase chain reaction can be analyzed by the use of denaturing gradient gel electrophoresis. Different alleles can be identified based on the different sequence-dependent melting properties and electrophoretic migration of DNA in solution. DNA molecules melt in segments, termed melting domains, under conditions of increased temperature or denaturation.

Each melting domain melts cooperatively at a distinct, base-specific melting temperature (Tm). Melting domains are at least 20 base pairs in length, and can be up to several hundred base pairs in length.

Differentiation between alleles based on sequence specific melting domain differences can be assessed using polyacrylamide gel electrophoresis, as described in Chapter 7 of Erlich, ed., PCR Technology, Principles and Applications for DNA Amplification, W. H. Freeman and Co., New York (1992).

Generally, a target region to be analyzed by denaturing gradient gel electrophoresis is amplified using PCR primers flanking the target region. The amplified PCR product is applied to a polyacrylamide gel with a linear denaturing gradient as described in Myers et al., Meth. Enzymol. 155:501-527, 1986, and Myers et al., in Genomic Analysis, A Practical Approach, K. Davies Ed. IRL Press Limited, Oxford, pp. 95 139, 1988. The electrophoresis system is maintained at a temperature slightly below the Tm of the melting domains of the target sequences.

In an alternative method of denaturing gradient gel electrophoresis, the target sequences can be initially attached to a stretch of GC nucleotides, termed a GC clamp, as described in Chapter 7 of Erlich, supra. In one example, at least 80% of the nucleotides in the GC clamp are either guanine or cytosine. In another example, the GC clamp is at least 30 bases long. This method is particularly suited to target sequences with high Tm's.

Generally, the target region is amplified by the polymerase chain reaction as described above. One of the oligonucleotide PCR primers carries at its 5' end, the GC clamp region, at least 30 bases of the GC rich sequence, which is incorporated into the 5' end of the target region during amplification. The resulting amplified target region is run on an electrophoresis gel under denaturing gradient conditions as described above. DNA fragments differing by a single base change will migrate through the gel to different positions, which can be visualized by ethidium bromide staining.

### Temperature Gradient Gel Electrophoresis

Temperature gradient gel electrophoresis (TGGE) is based on the same underlying principles as denaturing gradient gel electrophoresis, except the denaturing gradient is produced by differences in temperature instead of differences in the concentration of a chemical denaturant. Standard TGGE utilizes an electrophoresis apparatus with a temperature gradient running along the electrophoresis path. As samples migrate through a gel with a uniform concentration of a chemical denaturant, they encounter increasing temperatures. An alternative method of TGGE, temporal temperature gradient gel electrophoresis (TTGE or tTGGE) uses a steadily increasing temperature of the entire electrophoresis gel to achieve the same result. As the samples migrate through the gel the temperature of the entire gel increases, leading the samples to encounter increasing temperature as they migrate through the gel. Preparation of samples, including PCR amplification with incorporation of a GC clamp, and visualization of products are the same as for denaturing gradient gel electrophoresis.

### Single-Strand Conformation Polymorphism Analysis

Target sequences or alleles can be differentiated using single-strand conformation polymorphism analysis, which identifies base differences by alteration in electrophoretic migration of single stranded PCR products, for example as described in Orita et al., Proc. Nat. Acad. Sci. 85:2766-2770, 1989. Amplified PCR products can be generated as described above, and heated or otherwise denatured, to form single stranded amplification products. Single-stranded nucleic acids can refold or form secondary structures which are partially dependent on the base sequence. Thus, electrophoretic mobility of single-stranded amplification products can detect base-sequence difference between alleles or target sequences.

### Chemical or Enzymatic Cleavage of Mismatches

Differences between target sequences can also be detected by differential chemical cleavage of mismatched base pairs, for example as described in Grompe et al., Am. J. Hum. Genet. 48:212-222, 1991. In another method, differences between target sequences can be detected by enzymatic cleavage of mismatched base pairs, as described in Nelson et al., Nature Genetics 4:11-18, 1993. Briefly, genetic material from an animal and an affected family member can be used to generate mismatch free heterohybrid DNA duplexes. As used herein, 'heterohybrid' means a DNA duplex strand comprising one strand of DNA from one animal, and a second DNA strand from another animal, usually an animal differing in the phenotype for the trait of interest.

### Non-gel Systems

Other possible techniques include non-gel systems such as TaqMan™ (Perkin Elmer). In this system oligonucleotide PCR primers are designed that flank the mutation in question and allow PCR amplification of the region. A third oligonucleotide probe is then designed to hybridize to the region containing the base subject to change between different alleles of the gene. This probe is labelled with fluorescent dyes at both the 5' and 3' ends. These dyes are chosen such that while in this proximity to each other the fluorescence of one of them is quenched by the other and cannot be detected. Extension by Taq DNA polymerase from the PCR primer positioned 5' on the template relative to the probe leads to the cleavage of the dye attached to the 5' end of the annealed probe through the 5' nuclease activity of the Taq DNA polymerase. This removes the quenching effect allowing detection of the fluorescence from the dye at the 3' end of the probe. The discrimination between different DNA sequences arises through the fact that if the hybridization of the probe to the template molecule is not complete, i.e. there is a mismatch of some form, the cleavage of the dye does not take place. Thus only if the nucleotide sequence of the oligonucleotide probe is completely complimentary to the template molecule to which it is bound will quenching be removed. A reaction mix can contain two different probe sequences each designed against different alleles that might be present thus allowing the detection of both alleles in one reaction.

Many current methods for the detection of allelic variation are reviewed by Nollau et ah, Clin. Chem. 43, 1114-1120, 1997; and in standard textbooks, for example "Laboratory Protocols for Mutation Detection", Ed. by U. Landegren, Oxford University Press, 1996 and "PCR", 2nd Edition by Newton & Graham, BIOS Scientific Publishers Limited, 1997.

### SAMPLE

The test sample of nucleic acid is conveniently a sample of blood, mouth swab, biopsy, or other body fluid or tissue obtained from an individual. It will be appreciated that the test sample may equally be a nucleic acid sequence corresponding to the sequence in the test sample, that is to say that all or a part of the region in the sample nucleic acid may firstly be amplified using any convenient technique e.g. PCR, before analysis of allelic variation. The invention includes the detection of the polymorphism determined from a nucleic acid sample (which may be as defined above) that has already been removed from the individual.

### OMEGA-3 POLYUNSATURATED FATTY ACID

Polyunsaturated fatty acids (PUFA) can be classified into two major families (depending on the position (n) of the first double bond nearest the methyl end of the fatty acid carbon chain). Thus, the omega-6 fatty acids have the first unsaturated double bond six carbon atoms from the omega (methyl) end of the molecule and additionally have a total of two or more double bonds, with each subsequent unsaturation occurring 3 additional carbon atoms toward the carboxyl end of the molecule. In contrast, the omega-3 fatty acids have the first unsaturated double bond three carbon atoms away from the omega end of the molecule and additionally have a total of three or more double bonds, with each subsequent unsaturation occurring 3 additional carbon atoms toward the carboxyl end of the molecule.

Table 4 summarizes the common names of omega-3 fatty acids and the abbreviations that will be used throughout the specification:

**Table 4**

| Common Name | Abbreviation | Shorthand notation |
|---|---|---|
| oleic acid | OA | 18:1^{Δ9} |
| Linoleic acid | LA | 18:2^{Δ9,12} |
| γ-Linolenic | GLA | 18:3^{Δ6,9,12} |
| di-homo γ-linolenic acid | DGLA | 20:3^{Δ8,11,14} |
| Arachidonic acid | ARA | 20:4^{Δ5,8,11,14} |
| α-linolenic acid | ALA | 18:3^{Δ9,12,15} |
| stearidonic acid | SDA | 18:4 ^{Δ6,9,12,15} |
| eicosatetraenoic acid | ETA | 20:4 ^{Δ8,11,14,17} |
| eicosapentaenoic acid | EPA | 20:5^{Δ5,8,11,14,17} |
| docosapentaenoic acid | DPA | 22:5^{Δ7,10,13,16,19} |
| docosahexaenoic acid | DHA | 22:6^{Δ4,7,10,13,16,19} |

The three main types of omega-3 PUFA involved in human physiology are α-linolenic acid (ALA) (found in plant oils), eicosapentaenoic acid (EPA), and docosahexaenoic acid (DHA) (both commonly found in marine oils). Currently the primary dietary source of omega-3 highly unsaturated fatty acids is from certain fish oils which can contain up to 20-30% of these fatty acids in their triacylglycerides. Consequently large quantities of fish oil are processed and encapsulated each year for sale as a dietary supplement. Common sources of plant oils containing ALA include walnut, edible seeds, clary sage seed oil, algal oil, flaxseed oil, Sacha Inchi oil, Echium oil, and hemp oil. Sources of EPA and DHA include fish oils, egg oil, squid oils, and krill oil.

The omega-3 PUFA may be provided in small amounts of oils containing high quantities of preformed omega-3 PUFA, such as fish oils or microbial oils.

In one embodiment, the omega-3 PUFA is selected from EPA (C20:5, omega-3) and DHA (C22:6, omega-3) or a mixture thereof.

The omega-3 PUFA, for example as provided in a nutritional composition as described herein, may comprise an EPA to DHA ratio of about 0.5:1 to 5:1, 1:1 to 3:1 or 1.5:1 to 2.5:1 wt/wt. The omega-3 PUFA may comprise an EPA to DHA ratio of about 0.5:1, 1:1, 1.5:1, 2:1, 2.5:1, 3:1 or 5:1 wt/wt. In one embodiment the omega-3 PUFA comprises an EPA to DHA ratio of about 2.5:1 wt/wt.

An example of a commercial source of omega-3 PUFA is GNC Preventive Nutrition^{®} Triple Strength Fish Oil.

### DOSAGE

The actual dosage of omega-3 PUFA may be varied to provide a dose which will be most suitable for an individual subject. The dose may vary with the age, weight and response of the particular subject.

The subject may, for example, be administered from about 500 to about 10000 mg omega-3 PUFA per day. In one embodiment the subject is administered from about 1500 to about 3000 mg omega-3 PUFA per day.

The subject may, for example, be administered about 500 to 4000 mg EPA per day. In one embodiment the subject is administered about 1000 to 3000 mg EPA per day. In one embodiment the subject is administered about 2000 mg EPA per day.

The subject may, for example, be administered about 500 to 4000 mg DHA per day. In one embodiment the subject is administered about 500 to 3000, 500 to 2000 or 500 to 1000 mg DHA per day. In one embodiment the subject is administered about 500 to 1000 mg DHA per day. In one embodiment the subject is administered about 750 mg DHA per day.

The subject may, for example, be administered about 500 to 4000 mg EPA and about 500 to 4000 mg DHA per day. In one embodiment the subject is administered about 1000 to 3000 mg EPA and about 500 to 1000 mg DHA per day. In one embodiment the subject is administered about 2000 mg EPA and about 750 mg DHA per day.

### NUTRITIONAL COMPOSITION

The term nutritional composition means a composition which nourishes a subject. The nutritional composition is usually to be taken orally, intragastrically or intravenously. Preferably, the nutritional composition for use in the present invention is to be taken orally.

Nutritional compositions, as used herein, may include any number of optional ingredients in addition to omega-3 PUFA. Such additional ingredients include, but are not limited to, conventional food additives (synthetic or natural), for example one or more acidulants, additional thickeners, buffers or agents for pH adjustment, chelating agents, colorants, emulsifies, excipient, flavour agent, mineral, osmotic agents, a pharmaceutically acceptable carrier, preservatives, stabilizers, sugar, sweeteners, texturizers, and/or vitamins. The optional ingredients can be added in any suitable amount.

The nutritional composition may be in the form of powder, tablets, capsules, pastilles or a liquid for example. The composition may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins etc.), adsorbents, carriers, fillers, co-compounds, dispersing agents, wetting agents, processing aids (solvents), flowing agents, taste masking agents, weighting agents, jellifying agents and gel forming agents. The composition may also contain conventional pharmaceutical additives and adjuvants, excipients and diluents, including, but not limited to, water, gelatine of any origin, vegetable gums, lignin-sulfonate, talc, sugars, starch, gum arabic, vegetable oils, polyalkylene glycols, flavouring agents, preservatives, stabilizers, emulsifying agents, buffers, lubricants, colorants, wetting agents, fillers, and the like.

The composition may be usable for reconstitution in water. The composition may be an oil-in-water emulsion.

The nutritional composition may contain a carbohydrate source in addition to the omega-3 PUFA.

In one embodiment, at least 70, 80, 90, 95, 98, 99 or 100% of the PUFA in the nutritional composition are omega-3 PUFA. In one embodiment the nutritional composition does not comprise omega-6 PUFA.

The nutritional composition may contain vitamins and minerals understood to be essential in the daily diet and in nutritionally significant amounts. Minimum requirements have been established for certain vitamins and minerals. Examples of minerals, vitamins and other nutrients optionally present in the composition include vitamin A, vitamin B1, vitamin B2, vitamin B6, vitamin B12, vitamin E, vitamin K, vitamin C, vitamin D, folic acid, inositol, niacin, biotin, pantothenic acid, choline, calcium, phosphorous, iodine, iron, magnesium, copper, zinc, manganese, chlorine, potassium, sodium, selenium, chromium, molybdenum, taurine, and L- carnitine. Minerals are usually added in salt form. The presence and amounts of specific minerals and other vitamins will vary depending on the intended population.

The nutritional composition may also contain other substances which may have a beneficial effect such as lactoferrin, nucleotides, nucleosides, gangliosides, polyamines, phytosterols, fibres and the like.

The nutritional composition may be in the form of a nutritional supplement. A nutritional supplement refers to a product which is intended to supplement the general diet of a subject.

The composition may be in the form of a complete nutritional product. A complete nutritional product refers to a product which is intended to be the sole item or meal or diet consumed by a subject. As such, a complete nutritional product may contain sufficient types and levels of macronutrients (proteins, fats and carbohydrates) to be sufficient to be a sole source of nutrition for the subject to which it is being administered.

The composition may be inserted or mixed into a food substance. The composition may be in the form of a food stuff, for example a human food stuff.

The nutritional composition may be in the form of a tablet or capsule.

The nutritional composition may be enriched with omega-3 PUFA. 'Enriched' means that omega-3 PUFA has been added to the composition. For example, omega-3 PUFA may be spiked (i.e. added within or into) the composition.

In one embodiment, where a nutritional composition natively contains omega-3 PUFA, enriched with omega-3 PUFA means that the enriched composition comprises a greater amount of the compound than occurs naturally natively in the composition.

For example, an enriched composition may comprise at least 1.5-, at least 2-, at least 5-, at least 10-, at least 20-, at least 50- or at least 100-fold more omega-3 PUFA than an equivalent naturally occurring native composition which has not been enriched.

Enrichment may be achieved by adding omega-3 PUFA to the composition, for example by spraying or spiking it with omega-3 PUFA.

### SUBJECT

The subject may be, but is not limited to, mammals such as bovine, canine, caprine, cervine, equine, feline, human, ovine, porcine and primates. The subject may be a companion animal. Preferably, the subject is a human. In various embodiments, the subject may have, or be suspected of or at risk of, a condition associated with increased fasting TAG levels.

A condition associated with elevated TAG levels includes hypertriglyceridemia, hyperlipidaemia, dyslipidaemia, cardiovascular disease, atherosclerosis, atheroma, acute pancreatitis, type 2 diabetes, metabolic syndrome, hypertension and non-alcoholic fatty liver disease.

The subject may be at risk of developing, have a predisposition for or be suffering from elevated TAG levels.

The subject may be at risk of developing, have a predisposition for or be suffering from atherosclerosis.

The subject may be at risk of developing, have a predisposition for or be suffering from hypertriglyceridemic and/or hyperlipidaemic.

### USE

In one aspect, the present invention also provides the use of an omega-3 PUFA or a nutritional composition comprising an omega-3 PUFA as an active agent for reducing fasting TAG levels in a subject having a PPARα X162V genetic variant, where X is leucine or valine.

The nutritional composition may be any nutritional composition as described herein.

In another aspect, the present invention provides an omega-3 PUFA or a nutritional composition comprising an omega-3 PUFA as an active agent for use in reducing TAG levels in a subject previously identified as having a PPARα X162V genetic variant, where X is leucine or valine.

The subject may be suffering from a condition referred to herein which is associated with elevated TAG levels.

In a further aspect, the present invention provides an omega-3 PUFA or a nutritional composition comprising an omega-3 PUFA as an active agent for use in reducing TAG levels to prevent or treat a condition associated with elevated TAG levels in a subject previously identified as having a PPARα X162V genetic variant, where X is leucine or valine.

In preferred embodiments, the subject also has a PPARy2 X'12A genetic variant; where X' is proline or alanine.

### METHOD

In one aspect the present invention relates to a method for reducing TAG levels in a subject comprising administering an omega-3 PUFA or a nutritional composition comprising an omega-3 PUFA as an active agent to the subject; wherein the subject has been identified as having a PPARα X162V genetic variant, where X is leucine or valine.

The subject may be suffering from a condition referred to herein which is associated with elevated TAG levels.

In one aspect the present invention relates to a method for reducing TAG levels to prevent or treat a condition associated with elevated TAG levels in a subject comprising administering an omega-3 PUFA or a nutritional composition comprising an omega-3 PUFA as an active agent to the subject; wherein the subject has been identified as having a PPARα X162V genetic variant, where X is leucine or valine.

The present methods comprise administering an effective amount of an omega-3 PUFA to the subject. An effective amount refers to an amount which is capable of, for example, reducing TAG levels in the subject.

The effective amount may differ depending on the weight, age or sex of the subject.

The present methods may comprise the steps of: i) determining whether the subject has a PPARα X162V genetic variant, where X is leucine or valine; and optionally determining whether the subject has a PPARα X162V genetic variant, where X' is proline or alanine and ii) administering an omega-3 PUFA or a nutritional composition comprising an omega-3 PUFA as an active agent to a subject identified has having a PPARα X162V genetic variant, where X is leucine or valine.

Determining the presence of a genetic variant may be performed using standard methods in the art such as those described herein.

In a further aspect, the invention may provide a method for determining whether a subject is susceptible to benefit from the consumption of PUFA comprising the steps of: a) determining whether the subject is positive for a PPARα X162V genetic variant and b) determining if the subject is susceptible to benefit from the consumption of PUFA (for example as a nutritional composition comprising an omega-3 PUFA as an active agent) depending of the carried genetic variant, wherein such benefit is related to the reduction of triglyceride levels.

Those skilled in the art will understand that they can freely combine all features of the present invention described herein, without departing from the scope of the invention as disclosed.

Various preferred features and embodiments of the present invention will now be described by way of non-limiting examples.

### EXAMPLES

### Example 1 - Impact of omega-3 PUFA supplementation on TAG levels

The bioefficacy of omega-3 PUFA supplementation (provided by intake of fish oil supplements) on fasting TAG levels was assessed.

As a primary outcome, changes blood plasma TAG level between baseline and six weeks after intervention were measured.

The present inventors determined that individuals carrying PPARα L162V and PPARy2 P12A genetic variant alleles had a greater reduction in fasting TAG levels compared to individuals homozygous for PPARα Leu162 and PPARy2 Pro12 following a six week omega-3 PUFA supplementation (see Table 3).

In particular, the results of the present study demonstrated that a six week supplementation with omega-3 PUFA (performed as outlined in the *Materials and Methods*) resulted in a reduction of fasting TAG levels of approximately 30% in individuals carrying the PPARα Leu162Val variant and the PPARy2 Pro12Ala variant (subgroup X162V/X12A) compared to approximately 3% in individuals homozygous for both wild-type alleles (subgroup L162L/P12P).

### Results

Following 6 weeks omega-3 PUFA supplementation the total combined study population exhibited a decrease blood plasma TAG levels of around 5% (see Figure 1 and Table 1).

With regard to PPARα genotype, individuals having a PPARα L162V genetic variant (subgroup X162V) exhibited a mean decrease in fasting TAG level of around 16%. In contrast, TAG levels in homozygous PPARα L162L individuals, i.e. those lacking the PPARα L162V genetic variant, were only decreased by around 4%.

**Table 1 - Fasting serum TAG response to omega-3 PUFA supplementation - PPARα**

| Triglycerides (mg/dL) | | Baseline | Week 6 | Change (Week 6-Baseline) | %Change ((Week 6/Baseline)-1)*100 |
|---|---|---|---|---|---|
| ALL | Mean | 97.919 (± 53.542) | 93.850 (± 57.926) | -4.069 (± 51.678) | -5.311 |
| N=191 | Median | 85.000 | 80.540 | -3.760 | -4.774 |
| | | | | | |
| PPARα L162L | Mean | 97.302 (± 53.953) | 95.138 (± 60.137) | -2.164 (±52.724) | -3.874 |
| N=170 | Median | 83.015 | 81.640 | -3.635 | -4.770 |
| | | | | | |
| **PPARα X162V** | **Mean** | **102.908 (± 51.060)** | **83.424 (± 34.48)** | **-19.484 (± 40.013)** | **-16.181** |
| **N=21** | **Median** | **93.640** | **70.000** | **-8.630** | **-15.601** |

Similarly, blood plasma TAG levels in those individuals having a PPARy2 P12A genetic variant (subject group X12A) decreased by around 11%. Homozygous PPARy2 P12P individuals, i.e. those lacking the PPARy2 P12A genetic variant, exhibited a TAG decrease of only around 4% (see Table 2 and Figure 1).

**Table 2 - Fasting serum TAG response to omega-3 PUFA supplementation - PPARγ2**

| Triglycerides (mg/dL) | | Baseline | Week 6 | Change (Week 6-Baseline) | %Change ((Week 6/Baseline)-1)*100 |
|---|---|---|---|---|---|
| ALL | Mean | 97.919 (± 53.542) | 93.850 (± 57.926) | -4.069 (± 51.678) | -5.311 |
| N=191 | Median | 85.000 | 80.540 | -3.760 | -4.774 |
| | | | | | |
| PPARγ2 P12P | Mean | 96.710 (± 50.123) | 95.161 (± 61.324) | -1.550 (± 53.696) | -3.936 |
| N=153 | Median | 85.000 | 84.870 | -3.570 | -4.278 |
| | | | | | |
| **PPARy2 X12A** | **Mean** | **102.784 (± 66.106)** | **88.573 (± 41.781)** | **-14.212 (± 41.679)** | **-10.651** |
| **N=38** | **Median** | **86.480** | **77.750** | **-8.330** | **-11.944** |

When combinations of both PPARα and PPARy2 genotypes were studied, it was found that L162L/P12P individuals (i.e. those lacking either PPAR genetic variant) demonstrated a small percentage decrease in TAG level, of around 3% (see Table 3). In line with the single genotype results, TAG levels in the L162L/X12A and X162V/P12P subject groups decreased by around 8% and 12%, respectively. However, surprisingly it was found that omega-3 PUFA supplementation of those individuals determined to have both PPARα L162V and PPARy2 P12A genetic variants resulted in a TAG decrease of around 30% (see Table 3 - X162V/X12A subject group). This percentage decrease was largest of all the participant subgroups.

**Table 3 - Fasting serum TAG response to omega-3 PUFA supplementation - PPARα and PPARγ2 combinations**

| Triglycerides (mg/dL) | | Baseline | Week 6 | Change (Week 6-Baseline) | %Change ((Week 6/Baseline)-1)*100 |
|---|---|---|---|---|---|
| PPARα L162L/PPARγ2 P12P | Mean | 97.736 (± 51.161) | 97.455 (± 63.520) | -0.281 (± 55.296) | -2.864 |
| N=137 | Median | 85.000 | 86.740 | -3.570 | -4.766 |
| | | | | | |
| PPARα L162L/PPARγ2 X12A | Mean | 95.501 (± 65.158) | 85.519 (± 42.813) | -9.982 (± 40.092) | -7.958 |
| N=33 | Median | 80.990 | 74.750 | -6.900 | -7.193 |
| | | | | | |
| PPARα X162V/PPARγ2 P12P | Mean | 87.924 (± 40.435) | 75.516 (± 32.644) | -12.408 (± 45.690) | -12.648 |
| N=16 | Median | 78.045 | 67.140 | -3.850 | -3.689 |
| | | 150.858 | 108.730 | | |
| PPARα X162V/PPARγ2 X12A | Mean | (± 55.966) | (± 29.982) | -42.128 (± 45.690) | -26.556 |
| N=5 | Median | 136.320 | 104.610 | -59.360 | -36.202 |

### Materials and methods

### Study participants

191 healthy male and female subjects between 18 to 40 years of ages, with a BMI between 18.5 and 30, non-smokers, with sedentary to moderate physical activity and no consumption of dietary supplements or any medication that can affect the study outcome.

### Omega-3 PUFA dosage

The participants' diets were supplemented with omega-3 PUFA at a dose of 2700 mg day, as provided by three fish oil capsules (GNC Preventive Nutrition^{®} Triple Strength Fish Oil), to be orally administered each day. Each capsule contains 647 mg eicosapentaenoic acid (EPA) and 253 mg docosahexaenoic acid (DHA).

### Study procedure

Participants visited the clinical setting on three occasions, three weeks apart. After a screening interview, potential participants were invited to the study site for their first visit during which they provided informed consent. After verification of the inclusion/exclusion criteria, enrolled participants undertook the following tests: clinical history, food frequency questionnaire, validated physical activity questionnaire, blood pressure measurement, anthropometric measurements (weight, height and waist circumference), body composition by bioelectric impedance using the InBody720 equipment (Biospace Co, Ltd.). A 12.5 ml blood sample was drawn under fasting conditions (> 8 hrs) for measurements of HbA1c %, glucose, insulin, triglycerides, total cholesterol, lipoprotein fractions, adiponectin and CRP in plasma and separation of PBMC for RNA and DNA isolation. 2.5 ml EDTA-blood was drawn for determination of fatty acid in the plasma membrane of erythrocytes and 10 ml for PBMC transcriptomics. Participants were also provided with the supplements for the next three weeks.

During a second visit, the results of HbA1c %, glucose, lipids, insulin and body composition were discussed by a nutritionist with the subjects. The subjects returned a side effects journal, filled out a 24 hour food recall questionnaire and provided with the supplements for the last three weeks.

During the second visit, the participants discussed the results of the HbA1c%, glucose, lipids, insulin and body composition tests with a nutritionist. Participants also returned return the side effects journal, filled out a 24 hr food recall questionnaire and were provided with the supplements for the last three weeks. The last visit, after six weeks supplementation, had the same parameters as the first visit, except that the clinical history and SNUT food frequency questionnaire were recorded. The 24 hr food recall questionnaire and physical activity questionnaires were collected on all three visits.

All publications mentioned in the above specification are herein incorporated by reference. Various modifications and variations of the described methods of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

## Claims

1. A nutritional composition comprising an omega-3 PUFA as an active agent for use in reducing triglyceride levels in a subject having a peroxisome proliferator-activated receptor-alpha (PPARα) X162V genetic variant; where X is leucine or valine.

2. A nutritional composition comprising an omega-3 PUFA as an active agent for use in reducing triglyceride levels to prevent or treat a condition associated with elevated triglyceride levels in a subject having a PPARα X162V genetic variant; where X is leucine or valine.

3. A nutritional composition for use according to claim 2 wherein the condition associated with elevated triglyceride levels is selected from hypertriglyceridemia, hyperlipidaemia, dyslipidaemia, cardiovascular disease, atherosclerosis, atheroma, acute pancreatitis, insulin resistance, prediabetes, type 2 diabetes, metabolic syndrome, hypertension and non-alcoholic fatty liver disease.

4. A nutritional composition for use according to any preceding claim wherein the subject also has a proliferator-activated receptor-gamma 2 (PPARy2) X'12A genetic variant; where X' is proline or alanine.

5. A nutritional composition for use according to any preceding claim wherein the subject is administered from about 500 to about 10000 mg omega-3 PUFA per day, preferably from about 1500 to about 3000 mg omega-3 PUFA per day.

6. A nutritional composition for use according to any preceding claim wherein the omega-3 PUFA comprises eicosapentaenoic acid (EPA) or docosahexanoic acid (DHA) or a mixture of EPA and DHA.

7. A nutritional composition for use according to claim 6 wherein the omega-3 PUFA comprises an EPA to DHA ratio of about 0.5:1to about 5:1 wt/wt; preferably about 2.5:1 wt/wt.

8. A nutritional composition for use according to claim 7 wherein the subject is administered about 500 to 4000 mg EPA and about 500 to 4000 mg DHA per day, preferably about 1000 to 3000 mg EPA and about 500 to 1000 mg DHA per day.

9. A nutritional composition for use according to any preceding claim wherein the composition is in the form of a foodstuff, preferably a human food stuff.

10. A nutritional composition for use according to any of claims 1 to 8 wherein the composition is in the form of a complete nutritional product.

11. A nutritional composition for use according to any of claims 1 to 8 wherein the composition is in the form of a capsule, a tablet, a powdered form, a freeze-dried product or an oil-in-water emulsion.

12. A method for reducing triglyceride levels, or preventing or treating a condition associated with elevated triglyceride levels by reducing triglyceride levels in a subject comprising administering a nutritional composition comprising an omega-3 PUFA as an active agent to the subject; wherein the subject has been identified as having a PPARα X162V genetic variant; where X is leucine or valine.

13. A method according to claim 12 wherein the subject has also been identified as having a proliferator-activated receptor-gamma 2 (PPARy2) X'12A genetic variant; where X' is proline or alanine.

14. A method for reducing triglyceride levels, or preventing or treating a condition associated with elevated triglyceride levels by reducing triglyceride levels in a subject comprising the steps of:
i) determining whether the subject is positive for a PPARα X162V genetic variant; and
ii) administering a nutritional composition comprising an omega-3 PUFA as an active agent to a subject identified as having the PPARα X162V genetic variant; where X is leucine or valine.

15. Use of an omega-3 PUFA in the preparation of a nutritional product for reducing triglyceride levels, or preventing or treating a condition associated with elevated triglyceride levels by reducing triglyceride levels in a subject, wherein the subject has a PPARα X162V genetic variant; where X is leucine or valine.
